(19) Europäisches Patentamt
European Patent Office
Office européen des brevets

(11) Veröffentlichungsnummer: **0 377 197**
**A1**

# (12) EUROPÄISCHE PATENTANMELDUNG

(21) Anmeldenummer: **89123921.2**

(51) Int. Cl.[5]: **C07D 211/46, C08K 5/3435**

(22) Anmeldetag: **27.12.89**

(30) Priorität: **30.12.88 DE 3844356**

(43) Veröffentlichungstag der Anmeldung:
**11.07.90 Patentblatt 90/28**

(84) Benannte Vertragsstaaten:
**BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Aumueller, Alexander, Dr.**
**An der Marlach 5**
**D-6705 Deidesheim(DE)**
Erfinder: **Neumann, Peter, Dr.**
**Poststrasse 28**
**D-6800 Mannheim 31(DE)**
Erfinder: **Trauth, Hubert**
**Milanstrasse 5**
**D-6724 Dudenhofen(DE)**

## (54) 4-(2'-Ethylhexanoyloxy)-2,2,6,6-tetramethyl-piperidin und dessen Verwendung.

(57) Die Erfindung betrifft 4-(2'-Ethylhexanoyloxy)-2,2,6,6-tetramethyl-piperidin (Formel I) und dessen Verwendung als Mittel zur Stabilisierung von Kunststoffen und Lacken gegen Licht und Wärme.

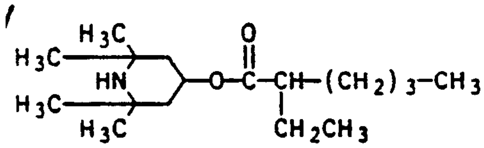

(I)

EP 0 377 197 A1

# 4-(2′-Ethylhexanoyloxy)-2,2,6,6-tetramethyl-piperidin und dessen Verwendung

Es ist bekannt, daß Tetramethylpiperidinderivate Polymere vor Abbau durch Licht, Sauerstoff und Wärme schützen. So sind beispielsweise in der DE-PS 1 929 928 Ester des 2,2,6,6-Tetramethylpiperidinols und in der DE-OS 2 258 752 1-substituierte Ester dieser Verbindung als Lichtschutzmittel beschrieben.

Die Verträglichkeit mit dem zu schützenden Polymeren sowie die Dauer und Güte der Lichtschutzwirkung dieser Verbindungen ist jedoch nicht in allen Fällen befriedigend.

Der Erfindung lag die Aufgabe zugrunde, einen neuen Stabilisator zur Verfügung zu stellen, der die vorstehenden Nachteile nicht aufweist.

Überraschend wird diese Aufgabe mit der neuen Piperidinverbindung gelöst.

Dementsprechend betrifft die Erfindung 4-(2′-Ethylhexanoyloxy)-2,2,6,6-tetramethylpiperidin (I) sowie dessen Säureadditionssalze.

Überraschenderweise erzielt man mit (I) im Vergleich zu den nächstliegenden bekannten Estern eine bessere Stabilisierung von Kunststoffen wie Polyurethanen oder Polyolefinen oder von Lacken.

Die erfindungsgemäße Verbindung läßt sich nach an sich literaturbekannten Verfahren herstellen, z.B. durch Veresterung von 2,2,6,6-Tetra-methyl-4-piperidinol (II) und 2-Ethylhexansäure (III) oder deren reaktionsfähigen Derivaten wie dem Säurechlorid oder durch Umesterung von (II) mit Estern von (III) mit niederen Alkoholen wie dem Methyl- oder Ethylester herstellen.

Bei der Veresterung des 2,2,6,6-Tetramethyl-4-piperidinols mit 2-Ethylhexansäurechlorid arbeitet man vorzugsweise in einem inerten organischen Lösungsmittel, z.B. Dichlormethan, Toluol, Essigsäureethylester, Essigsäurebutylester, Xylol, Ethylenglykoldimethyl-ether oder Benzoesäuremethylester. Man kann mit oder ohne Hilfsbase wie Triethylamin, Tributylamin oder Pyridin arbeiten. Arbeitet man in einem Zweiphasensystem aus Wasser und einem mit Wasser nicht mischbaren organischen, inerten Lösungsmittel, so kann man beispielsweise auch Natrium- oder Kaliumhydroxyd oder Natrium- oder Kaliumcarbonat als Hilfsbase verwenden. Man arbeitet dabei bei einer Temperatur zwischen -20 und 100 °C, vorzugsweise bei 0 bis 50 °C und insbesondere bei Temperaturen um Raumtemperatur.

Die Umsetzung von 2,2,6,6-Tetramethyl-4-piperidinol mit Estern der 2-Ethylhexansäure mit niederen Alkoholen wie den Methyl- und Ethylestern kann in Gegenwart oder vorzugsweise in Abwesenheit von Lösungsmittel ausgeführt werden. Dabei ist es vorteilhaft, bei Temperaturen von 150 °C bis 200 °C, vorzugsweise bei 175 bis 180 °C zu arbeiten. Vorteilhafterweise setzt man dem Reaktionsgemisch einen Katalysator beispielsweise Natriumethylat, Natriummethylat, Natriumhydroxyd oder ein Orthotitanat wie Tetrabutylorthotitanat oder Tetra-isopropyl-ortho-titanat zu.

Die erfindungsgemäße Verbindung (I) wird üblicherweise durch Destillation isoliert und gereinigt.

Die Verbindung (I) kann in Form der freien Base oder als Salz vorliegen. Geeignete Anionen stammen z. B. von anorganischen Säuren, Carbonsäuren und Sulfonsäuren. Von den Salzen sind die der Carbonsäuren und Sulfonsäuren bevorzugt.

Als anorganische Anionen kommen z. B. Chlorid, Bromid, Sulfat, Methosulfat, Tetrafluorborat, Phosphat und Rhodanid in Betracht.

Carbonsäure-Anionen sind beispielsweise: Formiat, Acetat, Propionat, Hexanoat, Cyclohexanoat, Lactat, Stearat, Palmitat, Dodecylbenzoat, Benzoat, Acrylat, Methacrylat, Zitrat, Malonat oder Succinat sowie Anionen von Polycarbonsäuren, wie Polyacrylsäure, Polymethacrylsäure und (Meth)-Acrylsäure enthaltenden Copolymeren mit bis zu 3000 COOH-Gruppen.

Sulfonsäure-Anionen sind beispielsweise Benzolsulfonat, Tosylat und Methylsulfonat.

Die Erfindung betrifft außerdem die Verwendung von (I) zum Stabilisieren von Kunststoffen und Lacken, insbesondere von Polyolefinen und Polyurethanen.

Den zu stabilisierenden Kunststoffen wird die Verbindung (I) in einer Konzentration von 0,01 bis 5 Gew.% vorzugsweise 0,02 bis 2 Gew.% vor, während oder nach der Polymerbildung zugesetzt.

Zur Herstellung der Gemische aus der erfindungsgemäßen Verbindung (I) und den zu stabilisierenden Kunststoffen können alle bekannten Verfahren zum Einmischen von Stabilisierungsmitteln oder anderen Zusätzen in Polymere angewandt werden.

Neben der erfindungsgemäßen Verbindung (I) können die stabilisierten Kunststoffe gegebenenfalls noch weitere Additive enthalten, beispielsweise Antioxidantien, zusätzliche Lichtstabilisierungsmittel, Metalldesaktivatoren, antistatische Mittel, flammhemmende Mittel, außerdem Pigmente und Füllstoffe.

Antioxidantien und Lichtstabilisatoren, die den Kunststoffen neben der erfindungsgemäßen Verbindung zugesetzt werden können, sind z.B. Verbindungen auf der Basis sterisch gehinderter Phenole, Schwefel und/oder Phosphor enthaltende Costabilisatoren.

Als derartige phenolische Antioxidationsmittel seien beispielsweise 2,6-Di-tert.-butyl-4-methylphenol, n-Octadecyl-ß-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[ß-(3,5-di-tert.-butyl-4-hydroxyphenyl)-propionyloxyethyl]-isocyanurat, 1,3,5-Tris-(2,6-di-methyl-3-hydroxy-4-tert.-butyl-benzyl)-isocyanurat und Pentaerythrit-tetrakis-[ß-(3,5-di-tert.-butyl-4-hydroxy-phenyl)-propionat] erwähnt.

Als phosphorhaltige Antioxidantien kommen z.B. Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-ditert.-butylphenyl)-pentaerythritdiphosphit und Tetrakis-(2,4-di-tert.-butylphenyl)-4,4′-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(ß-laurylthiopropionat) und Pentaerythrittetrakis-(ß-hexylthiopropionat) erwähnt.

Weitere Antioxidantien und Lichtstabilisatoren, die zusammen mit der erfindungsgemäßen Verbindung verwendet werden können, sind z.B. 2-(2′-Hydroxyphenyl)-benztriazole, 2-Hydroxybenzophenone, Phenylester von Hydroxybenzoesäuren, α-Cyanozimtsäurederivate, Nickelverbindungen, Benzimidazolcarbonsäureanilide und/oder Oxalsäuredianilide.

Als organische Polymere, die durch die erfindungsgemäße Verbindung stabilisiert werden können, kommen z. B. in Betracht:

Polymere von Mono- und Diolefinen, wie Polyethylen niedriger und hoher Dichte, lineares Polyethylen niedriger Dichte, Polypropylen, Polyisobutylen, Polybuten-1, Polyisopren, Polybutadien sowie Copolymerisate von Mono- oder Diolefinen und Mischungen der genannten Polymeren;

Copolymerisate von Mono- oder Diolefinen mit anderen Vinylmonomeren z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, z. B. Polyvinylchlorid, Polyvinylfluorid, Polyvinylidenfluorid sowie deren Copolymere;

Polymere die sich von α,ß-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.

Weitere organische Polymere, die mit der erfindungsgemäßen Verbindung stabilisiert werden können, sind Industrielackierungen, von denen die Einbrennlackierungen, von diesen wiederum Fahrzeuglackierungen, vorzugsweise Zweischichtlackierungen, besonders hervorzuheben sind. Auch hier können die bereits genannten weiteren Antioxidantien und Lichtschutzmittel noch zugesetzt werden.

Besonders bevorzugt ist die Verwendung der erfindungsgemäßen Verbindung in Polyurethanen und hier wiederum in Polyurethanen mit Polyetherolen als Polyolkomponente.

In Polyurethanen ist es zweckmäßig, eine Kombination aus

a) der erfindungsgemäßen Verbindung und mindestens eine Komponente aus den Gruppen

b) der UV-Absorber und/oder

c) der Antioxidantien aus den Klassen der sterisch gehinderten Phenole, der Phosphite oder des Vitamins E und seiner Derivate zu verwenden.

Die Stabilisatorkombination kann neben dem sterisch gehinderten Amin (I) auch noch andere sterisch gehinderte Amine in untergeordneten Mengen enthalten.

Als sterisch gehinderte Amine, die noch zusätzlich enthalten sein können, kommen z.B. in Betracht: Bis-(1,2,2,6,6-pentamethylpiperidyl)-ester wie beispielsweise Bis-(2,2,6,6-tetramethylpiperidyl)-sebacat, Bis-(1,2,2,6,6-pentamethylpiperidyl)-sebacat, das Kondensationsprodukt aus 1-Hydroxyethyl-2,2,6,6-tetramethyl-4-hydroxypiperidin und Bernsteinsäure, das Kondensationsprodukt aus N,N′-(2,2,6,6-Tetramethylpiperidyl)-hexamethylendiamin und 4-tert.-Octylamino-2,6-dichlor-1,3,5-s-triazin, Tris-(2,2,6,6-Tetramethylpiperidyl)-nitrilotriacetat, Tetrakis-(2,2,6,6-tetramethyl-4-piperidyl)1,2,3,4-butantetracarbonsäureester und 1,1′-(1,2-Ethandiyl)-bis-(3,3,5,5-tetramethylpiperazinon).

Für die Komponente (b) der UV-Absorber kommen z.B. solche aus den Klassen der Benzotriazole, der Formamidine und des N-Cyanoethylen-indolins in Betracht.

Im einzelnen sind für (b) z.B. zu nennen das 5′-Methyl-, 3′,5′-Di-tert.-butyl-, 5′-tert.-Butyl-, 5′-(1,1,3,3-Tetramethylbutyl)-, 5-Chlor-3′,5′-di-tert.-butyl-, 5-Chlor-3′-tert.-butyl-5′-methyl-, 3′-sec.-Butyl-5′-butyl-, 4′-Octoxy-, 3′,5′-Di-tert.-amyl- und/oder das 3′,5′-Bis(α,α-dimethylbenzyl)-Derivat des 2-(2′-Hydroxyphenyl)-benztriazols.

Als Indolinverbindung kommt z.B.

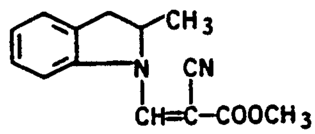

in Betracht.

Aus der Klasse der Formamidine sind beispielsweise die Verbindungen der Formeln

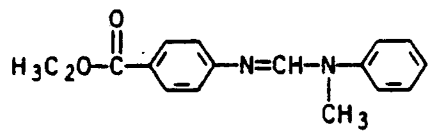

und

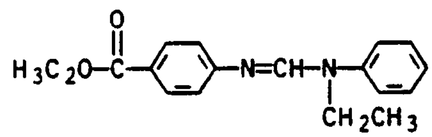

zu nennen.

Als Antioxidantien (Komponente (c)) kommen z.B. in Betracht:

c1) Phenolische Antioxidantien:

c1.1) alkylierte Monophenole,

wie z.B. 2,6-Di-tert.-butyl-4-methylphenol, 2-tert.-Butyl-4,6-dimethylphenol, 2,6-Ditert.-butyl-4-ethylphenol, 2,6-Di-tert.-butyl-4-n-butylphenol, 2,6-Di-tert.-butyl-4-i-butylphenol, 2,6-Di-cyclopentyl-4-methylphenol, 2-(α-methylcyclohexyl)-4,6-dimethylphenol, 2,6-Di-octadecyl-4-methylphenol, 2,4,6-Tri-cyclohexylphenol und 2,6-Di-tert.-butyl-4-methoxymethylphenol.

c1.2) alkylierte Hydrochinone,

z.B. 2,6-Di-tert.-butyl-4-methoxyphenol, 2,5-Di-tert.-butyl-hydrochinon, 2,5-Di-tert.-amylhydrochinon und 2,6-Di-phenyl-4-octadecyloxyphenol.

c1.3) hydroxylierte Thiodiphenylether,

wie z.B. 2,2′-Thio-bis-(tert.-butyl-4-methylphenol), 2,2′-Thio-bis-(4-octylphenol), 4,4′-Thio-bis-(6-tert.-butyl-3-methylphenol) und 4,4′-Thio-bis-(6-tert.-butyl-2-methylphenol).

c1.4) Alkyliden-Bisphenole,

z.B. 2,2′-Methylen-bis-(6-tert.-butyl-4-methylphenol),
2,2′-Methylen-bis-(6-tert.-butyl-4-ethylphenol),
2,2′-Methylen-bis-[4-methyl-6-8α-methylcyclohexyl)-phenol],
2,2′-Methylen-bis-(4-methyl-6-cyclohexyl)-phenol),
2,2′-Methylen-bis-(6-nonyl-4-methylphenol),
2,2′-Methylen-bis-(4,6-di-tert.-butylphenol),
2,2′-Ethyliden-bis-(4.6-di-tert.-butylphenol),
2,2′-Ethyliden-bis-(6-tert.-butyl-4-isobutylphenol),
2,2′-Methylen-bis-[6(α-methylbenzyl)-4-nonylphenol],
2,2′-Methylen-bis-[6-(α,α-dimethylbenzly)-4-nonylphenol],
4,4′-Methylen-bis-(2,6-di-tert.-butylphenol),
4,4′-Methylen-bis-(6-tert.-butyl-2-methylphenol),
1,1-bis-(5-tert.-butyl-4-hydroxy-2-methylphenyl)-butan,
2,6-Di-(3-tert.-butyl-5-methyl-2-hydroxybenzyl)-4-methylphenol,
1,1,3-Tris(5-tert.-butyl-4-hydroxy-2-methylphenyl)-butan,
1,1-Bis-(5-tert.-butyl-4-hydroxy-2-methylphenyl)-3-n-dodecylmercaptobutan,
Ethylenglykol-bis[3,3-bis-(3′-tert.-butyl-4′-hydroxyphenyl)-butyrat],

Di-(3-tert.-butyl-4-hydroxy-5-methylphenyl)-di-cyclopentadien und
Bis-[2-(3′-tert.-butyl-2′-hydroxy-5′-methylbenzyl)-6-tert.-butyl-4-methylphenyl]-terephthalat.

c1.5) Benzylverbindungen,

z.B.
1,3,5-Tris-(3,5-di-tert.-butyl-4-hydroxybenzyl)-2,4,6-trimethylbenzol,
Di-(3,5-di-tert.-butyl-4-hydroxybenzyl)-sulfid,
3,5-Di-tert.-butyl-4-hydroxy-benzyl-mercaptoessigsäure-isooctylester,
Bis-(4-tert.-butyl-3-hydroxy-2,6-dimethylbenzyl)-dithiolterephthalat,
1,3,5-Tris-(3,5-di-tert.-butyl-4-hydroxybenzyl)-isocyanurat,
1,3,5-Tris-(4-tert.-butyl-3-hydroxy-2,6-dimethylbenzyl)-isocyanurat,
3,5-Di-tert.-butyl-4-hydroxybenzyl-phosphonsäuredioctadecylester und
3,5-Di-tert.-butyl-4-hydroxybenzyl-phosphonsäure-monoethylester-Calcium-Salz.

c1.6) Acylaminphenole,

wie z.B. 4-Hydroxy-laurinsäureanilid,
4-Hydroxystearinsäureanilid,
2,4-Bis-octylmercapto-6-(3,5-di-tert.-butyl-4-hydroxyanilino)-s-triazin, und
N-(3,5-Di-tert.-butyl-4-hydroxyphenyl)-carbaminsäure-octylester.

c1.7) Ester der

ß-(3,5-Di-tert.-butyl-4-hydroxyphenyl)-propionsäure mit ein- oder mehrwertigen Alkoholen,
z.B. mit Methanol, Octadecanol, 1,6-Hexandiol, Neopentylglykol, Thiodiethylenglykol, Diethylenglykol, Triethylenglykol, Pentaerythrit, Tris-hydroxyethyl-isocyanurat und Di-hydroxyethyl-oxalsäurediamid.

c1.8) Ester der

ß-(5-tert.-Butyl-4-hydroxy-3-methylphenyl)-propionsäure mit ein- oder mehrwertigen Alkoholen,
z.B. mit Methanol, Octadecanol, 1,6-Hexandiol, Neopentylglykol, Thiodiethylenglykol, Diethylenglykol, Triethylenglykol, Pentaerythrit, Tris-hydroxyethyl-isocyanurat und Di-hydroxy-ethyl-oxalsäureamid.

c1.9) Amide der ß-(3,5-Di-tert.-butyl-4-hydroxyphenyl)-propionsäure,

z.B. N,N′-Di-(3,5-di-tert.-butyl-4-hydroxyphenylpropionyl)-hexame thylendiamin, N,N′-Di-(3,5-di-tert.-butyl-4-hydroxyphenylpropionyl)-trimet hylendiamin und N,N′-Di-(3,5-di-tert.-butyl-4-hydroxyphenylpropionyl)-hydrazin.

c2) Phosphorhaltige Antioxidationsmittel,

z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.-butyl-phenyl)-phosphit, Diisodecylpentaerythritdiphosphit, Di-(2,4-di-tert.-butylphenyl)-pentaerythritdiphosphit, Tristearyl-sorbit-triphosphit, Tetrakis-(2,4-di-tert.-butyl-phenyl)-4,4′-biphenylen-diphosphonit und 3,9-Bis-(2,4-di-tert.-butylphenoxy-2,4,8,10tetraoxa-3,9-diphosphaspiro[5,5] undecan.

c3) Vitamin E und dessen Derivate

z.B. Derivate der 6-Hydroxy-2,5,7,8-tetramethylchromans, wie α-Tocopherol, Stearinsäure-(6-hydroxy-2,5,7,8-tetramethylchroman-2-yl)-ethylester, Vitamin-E-acetat und -propionat.

Weiterhin können für (c) auch Mischungen gleichartiger und verschiedenartiger Antioxidantien in den erfindungsgemäßen Gemischen verwendet werden, z.B. Gemische aus α-Tocopherol und Tri-(nonylphenyl)-phosphit im Verhältnis von 1:10 bis 10:1 Gewichtsteilen.

Für (b) sind UV-Absorber aus der Klasse der 2-(2′-Hydroxyphenyl)-benztriazole und des N-Cyanoethylenindolins bevorzugt. Besonders bevorzugt sind für (b) das 5′-Methyl-, das 3′,5′-Di-tert.-butyl-, das 5′-tert.-Butyl-, das 3′,5′-Di-tert.-amyl, das 5-Chlor-3′,5′-di-tert.-butyl- und das 3′,5′-Bis(α,α-dimethylbenzyl)-Derivat des 2-(2′-Hydroxyphenyl)-benztriazols, die Verbindung der Formel

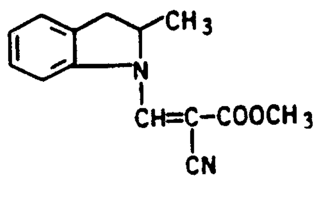

sowie eine Mischung aus Verbindungen der Formeln

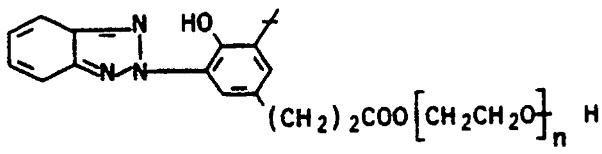

und

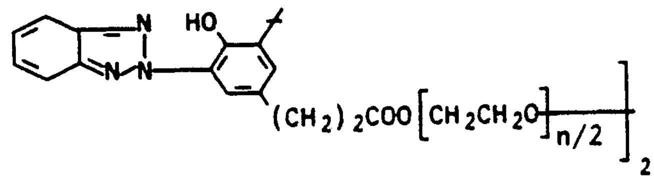

bei denen n so gewählt ist, daß das mittlere Molekulargewicht der Mischung ca. zwischen 600 und 1000 liegt.

Aus der Reihe der Antioxidantien sind 2,6-Di-tert.-butyl-4-methylphenol, 2-tert.-Butyl-4,6-dimethylphenol, Triethylenglykol-bis-3-(3-tert.-butyl-4-hydroxy-5-methylphenyl)-propionat, Pentaerythrit-tetr.-3-(3-tert.-butyl-4-hydroxy-5-methylphenyl)-propionat, α-Tocopherol, Stearinsäure-(6-hydroxy-2,5,7,8-tetramethyl-chroman-2-yl)-ethylester, Tri-(nonylphenyl)-phosphit und Tetra-bis-(2,4-di-tert.-butylphenyl)-4,4′-biphenylendiphosphonit bevorzugt.

Bevorzugt ist weiterhin eine Mischung aus α-Tocopherol und Tri-(nonylphenyl)-phosphit im Gewichtsverhältnis von 1:10 bis 10:1, insbesondere im Verhältnis von 1:10 Gewichtsteilen.

Die Komponenten (a), (b) und (c) der erfindungsgemäßen Stabilisatormischungen können als solche einzeln oder als Mischung dem Polyurethan in Mengen von jeweils 0,01 bis 5, vorzugsweise von jeweils 0,05 bis 2,5, insbesondere von jeweils 0,1 bis 2 Gew.%, bezogen auf das zu stabilisierende Material, zugemischt werden.

Üblicherweise erfolgt die Zugabe der Stabilisatormischung durch Lösen der einzelnen Verbindungen oder des Gemisches aus (a), (b) und/oder (c) in der Polyolkomponente des Polyurethans gegebenenfalls unter leichtem Erwärmen. Das erfindungsgemäße Gemisch kann aber auch durch Einmischen der einzelnen Verbindungen oder des fertigen Gemisches in die Schmelze nach der in der Technik üblichen Methode vor oder während der Formgebung eingebracht werden. Das Stabilisieren kann aber auch durch Aufbringen der gelösten oder dispergierten Verbindungen (a), (b) und/oder (c) auf das Polymere oder durch Einmischen in eine Lösung, Suspension oder Emulsion des Polymeren, gegebenenfalls unter nachträglichem Verdunsten des Lösungsmittels, erfolgen.

Die folgenden Beispiele sollen die Erfindung zusätzlich erläutern und verdeutlichen.

Herstellbeispiel

Zu einer Lösung von 139,9 g 2,2,6,6-Tetramethyl-4-hydroxypiperidin in 800 ml Dichlormethan tropfte man bei Raumtemperatur eine Lösung von 2-Ethylhexansäurechlorid in 200 ml Dichlormethan. Nach 3 Stunden Rühren bei Raumtemperatur wurde vom ausgefallenen 2,2,6,6-Tetramethyl-4-hydroxypiperidin-hydrochlorid abfiltriert, die Dichlormethanphase eingeengt und der Rückstand im Ölpumpenvakuum destilliert. Man erhielt 126 g der Verbindung der Formel

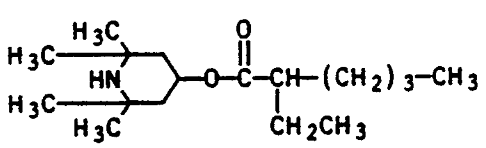

(I)

als farblose Flüssigkeit von Siedepunkt 101 °C/0,2 Torr.

| Ber.: | C 72,0 | H 11,7 | N 4,9 | O 11,3 % |
|---|---|---|---|---|
| Gef.: | C 72,1 | H 11,7 | N 5,2 | O 11,8 % |

Anwendungsbeispiel:

Stabilisierung von Polyurethanen

A) Herstellung der Proben für die Prüfung (Belichtung)

Eine Polyolkomponente, bestehend aus 41,9 Teilen eines Polyetherols (OH-Zahl: 29,0; erhalten durch Addition von Propylenoxid und Ethylenoxid an Propylenglykol und das ungefähr 84 % primäre Hydroxylgruppen besitzt), 42,5 Teile eines Polyetherols (OH-Zahl: 27,0; erhalten durch Addition von Propylenoxid und Ethylenoxid an Trimethylolpropan, das ungefähr 88 % pri märe Hydroxylgruppen besitzt), 8,1 Teilen 1,4-Butandiol, 1724 Teilen einer 25 %igen Lösung von Diazabicyclooctan in 1,4-Butandiol und 0,016 Teilen Dibutylzinndilaurat, 0,1 Teilen Siliconstabilisator OS 710 (Bayer AG), 5,49 Teilen Frigen 11 und 0,17 Teilen Wasser wurden mit den in der Tabelle angegebenen Stabilisatoren versetzt und im Mischungsverhältnis 100:48,5 Teilen mit einem 23,0 % Isocyanatgruppen enthaltenden Prepolymeren bei 25° C Komponenten- und Werkzeugtemperatur zu Prüfplatten verschäumt.

Das NCO-Prepolymere wurde hergestellt aus 87,17 Teilen 4,4′-Diphenylmethandiisocyanat und 4,83 Teilen eines Polyetherols mit der OH-Zahl von 250 (erhalten durch Addition von Propylenoxid an Propylenglykol) und 8,0 Teilen Dipropylenglykol.

B) Prüfung der Polyurethanproben

Die Proben werden im Xenotest®450 belichtet und an den Proben der Yellowness Index nach ASTM D 1925 bestimmt.

Die verwendeten Stabilisatorgemische sowie die Ergebnisse der Belichtung sind in der folgenden Tabelle zusammengestellt.

Tabelle 1

| Stabilisator | Menge (Gew.%) | Yellowness-Index nach ASTM D 1925 im Xenotest 450 nach 96 h |
|---|---|---|
| 0 | - | 49,4 |
| Gemisch aus | | |
| K 1, | 0,5 | |
| K 2 und | 0,5 | 15,3 |
| K 3 | 0,25 | |
| Gemisch aus | | |
| K 4, | 0,5 | |
| K 2 und | 0,5 | 17,5 |
| K 3 | 0,25 | |

K 1 = Verbindung des Herstellbeispiels K 2 = Gemisch aus

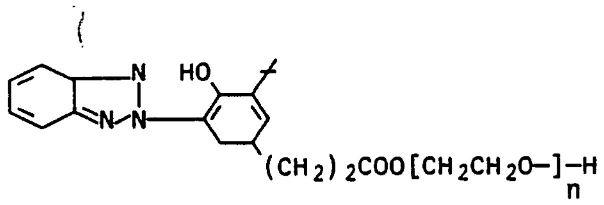

und

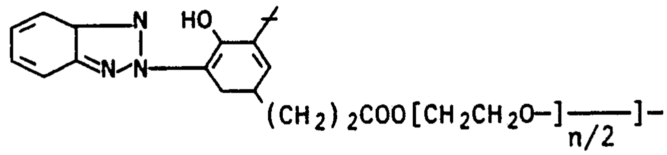

und

mittleres Molgewicht des Gemischs >600. K 3 = Mischung aus α-Tocopherol und Tri-(nonylphenyl)-phosphit (1:10 Gewichtsteile) K 4 = Verbindung der Formel

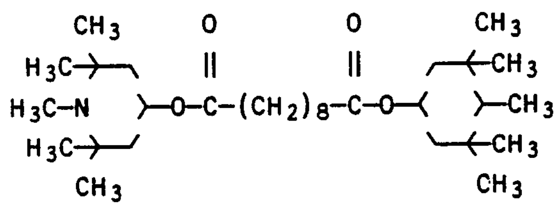

## Ansprüche

1. 4-(2′-Ethylhexanoyloxy)-2,2,6,6-tetramethylpiperidin sowie dessen Säureadditionssalze.

2. Verwendung der Piperidinverbindung gemäß Anspruch 1 zum Stabilisieren von Kunststoffen und Lacken.

3. Verwendung der Piperidinverbindung gemäß Anspruch 1 zum Stabilisieren von Polyurethanen oder Polyolefinen.

4. Verwendung der Piperidinverbindung gemäß Anspruch 1 zum Stabilisieren von Lacken.

Europäisches Patentamt

# EUROPÄISCHER RECHERCHENBERICHT

Nummer der Anmeldung

EP 89 12 3921

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X<br>Y | DE-A-2 258 752 (CIBA GEIGY)<br>* Seite 77, Beispiel 16; Seite 52, Formel IX; Seite 53, Zeilen 6-11; Ansprüche 1,118,175,176,182 *<br>--- | 1-4 | C 07 D 211/46<br>C 08 K 5/343 |
| Y | DE-A-1 929 928 (SANKYO CO.)<br>* Seite 3; Ansprüche 1,2,5,8 *<br>----- | 1-4 | |
| | | | **RECHERCHIERTE SACHGEBIETE (Int. Cl.5)** |
| | | | C 07 D 211/00 |

Der vorliegende Recherchenbericht wurde für alle Patentansprüche erstellt

| Recherchenort | Abschlußdatum der Recherche | Prüfer |
|---|---|---|
| DEN HAAG | 29-03-1990 | KISSLER B.E. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)